# EUROPEAN PATENT APPLICATION

(11) **EP 4 011 432 A1**
(43) Date of publication of application: **15.06.2022**
(21) Application number: 21213296.3
(22) Date of filing: 09.12.2021
(51) Int. Cl.: A61M 31/00, A61M 35/00, A61M 3/02, A45D 34/04, A61J 1/06

(54) **MEDICINE DISPENSING DEVICE**

(30) Priority: 10.12.2020 TW 109216304 U
(71) Applicant: Hu, Liujia, Taipei City 111 (TW)
(72) Inventor: Hu, Liujia, Taipei City 111 (TW)
(74) Representative: Cabinet Chaillot

(57) **Abstract**

A medicine dispensing device (1) includes a first tubular body (10) and a second tubular body (20). A removable section (12) is disposed at a dispensing end of the first tubular body (10) and a neck section (13) on the other end. A liquid medicine (111) and a stop member (112) are disposed in a receiving space (11) of the first body (10). At least one body girth of the stop member (112) is larger than the neck section (13). The second tubular body (20) includes a tube sealing section(21), a compression section (22) and a communication tube section (23) in connection with each other. The receiving space (11) is in communication with the interior of the second tubular body (20). The stop member (112) serves to block the neck section (13), so as to prevent the liquid medicine (111) from flowing into the second tubular body (20).

## Description

### BACKGROUND OF THE INVENTION

### 1. Field of the Invention

The present invention relates generally to a medicine dispensing device for dispensing a liquid medicine, and more particularly to a medicine dispensing device, which can prevent the liquid medicine from reversely flowing into or stagnating in an unexpected section of the medicine dispensing device.

### 2. Description of the Related Art

A female is often bothered with reproductive tract diseases and needs to see a doctor. Bacterial vaginitis and urethritis are both often seen infective diseases in gynecology (and obstetrics). The symptom of bacterial vaginitis and urethritis is pathogenic bacteria are over-reproduced in the vagina and urethra. In order to prevent and relieve vaginitis and urethritis and reduce the pathogenic bacteria in the vagina and urethra as well as enhance the anti-infective ability of the environment in the vagina and urethra, generally a vagina washing device is first used to dispense a liquid medicine or a suppository to kill the pathogenic bacteria and balance probiotics and pH value. Moreover, when a medical institution performs artificial reproduction or menopause treatment for a female, the medicine is often applied to the female by means of dispensing the medicine into the vagina. However, the conventional liquid medicine dispensing device for dispensing liquid medicine or suppository into the vagina is designed with a complicated structure so that the manufacturing cost of the conventional liquid medicine dispensing device is quite high and the fraction defective is increased in the manufacturing process. In addition, the conventional liquid medicine dispensing device has a common problem, that is, the product is subject to affection of transfer process or external force. As a result, the liquid medicine contained in the conventional liquid medicine dispensing device is apt to reversely flow into an unexpected section of the device. In this case, the device cannot be successfully used. Therefore, the conventional washing device or suppository still needs to improve.

It is therefore tried by the applicant to provide a medicine dispensing device, which can prevent the liquid medicine from reversely flowing into or stagnating in an unexpected section of the medicine dispensing device so as to eliminate the shortcomings existing in the conventional medicine dispensing device or suppository.

### SUMMARY OF THE INVENTION

It is therefore a primary object of the present invention to provide a medicine dispensing device including a first tubular body and a second tubular body. The first tubular body has an internal receiving space. A removable section is disposed at a dispensing end of the first tubular body. A liquid medicine and a stop member are disposed in the receiving space. A neck section is disposed at one end of the first tubular body distal from the removable section. The inner diameter of the neck section is smaller than the inner diameter of the first tubular body. At least one body girth (outer diameter or girth width) of the stop member is larger than the neck section, whereby the neck section can stop the stop member from passing through and the stop member can interrupt the internal passage of the neck section. The second tubular body includes a tube sealing section, a compression section for squeezing out the liquid medicine and a communication tube section sequentially in connection with each other. A rear end of the tube sealing section is a closed end. One end of the communication tube section, which end is distal from the tube sealing section, is in communication with the neck section, whereby the receiving space is in communication with the interior of the second tubular body. The stop member serves to block the neck section and interrupt the internal passage of the neck section so as to prevent the liquid medicine from flowing into or stagnating in the second tubular body in transfer or operation process. Therefore, the medicine dispensing device can be more smoothly used.

The present invention can be best understood through the following description and accompanying drawings, wherein:

### BRIEF DESCRIPTION OF THE DRAWINGS

Fig. 1 is a perspective view of the present invention;
Fig. 2 is a sectional view of the present invention;
Fig. 3 is a sectional view of the present invention, showing that a liquid medicine is contained in the present invention; and
Fig. 4 is a sectional view of the present invention, showing that the stop member interrupts the internal passage of the neck section to prevent the liquid medicine from flowing.

### DETAILED DESCRIPTION OF THE PREFERRED EMBODIMENTS

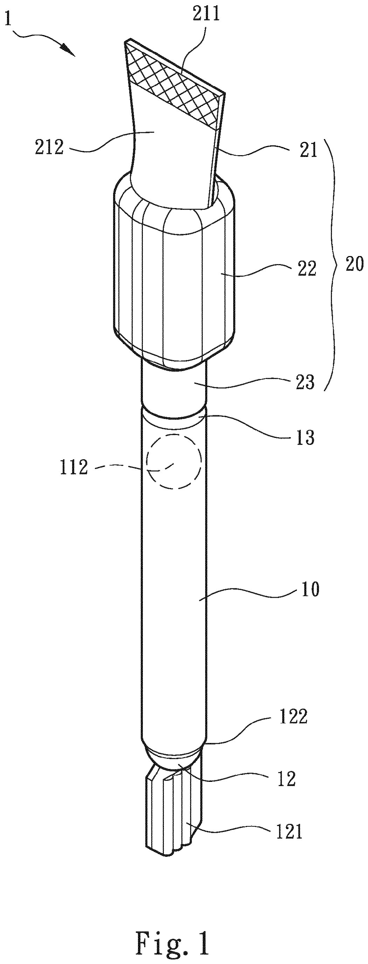

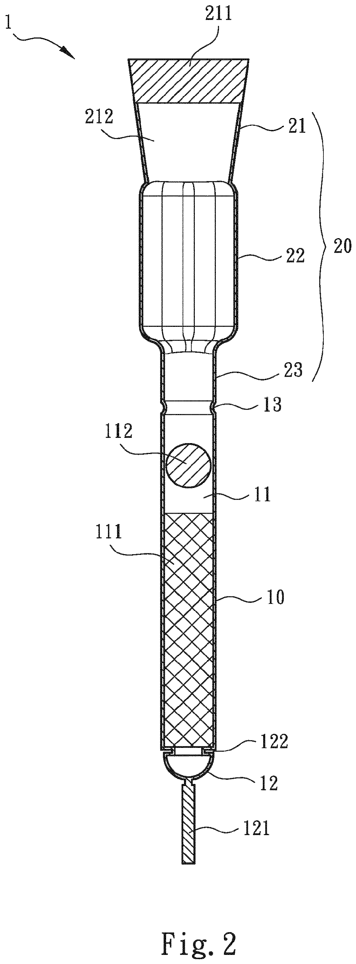

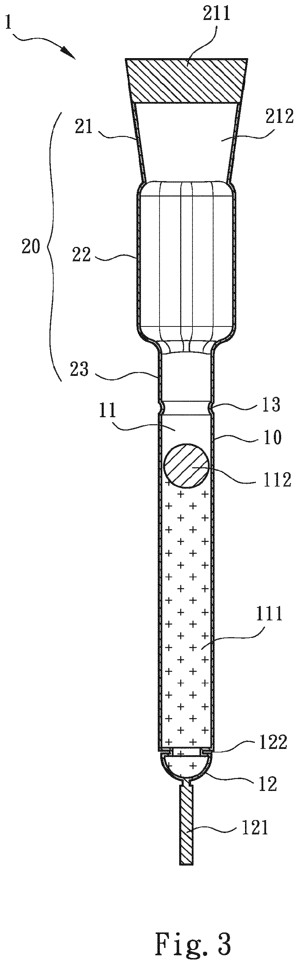

Please refer to Figs. 1 to 3. The medicine dispensing device 1 of the present invention includes a first tubular body 10 having an internal receiving space 11. A removable section 12 is disposed at a dispensing end of the first tubular body 10. A liquid medicine 111 and a stop member 112 are disposed in the receiving space 11. A first handheld section 121 is connected with a rear end of the removable section 12. The first handheld section 121 has a flat configuration. A breakable section 122 is disposed between the first tubular body 10 and the removable section 12, whereby the removable section 12 can be easily broken apart from the first tubular body 10. A user can pinch the first handheld section 121 and twist the same to easily detach the removable section 12 from the first tubular body 10. The liquid medicine 111 can be dispensed from the broken section of the first tubular body 10. According to the form classification, the liquid medicine 111 includes at least one of gel, cream and liquid medicine.

A neck section 13 is disposed at one end of the first tubular body 10 distal from the removable section 12. The neck section 13 has an inner diameter smaller than the inner diameter of the first tubular body 10. At least one body girth (such as outer diameter or girth width) of the stop member 112 is larger than the neck section 13, whereby the internal passage of the neck section 13 can be blocked and interrupted by the stop member 112. Accordingly, in transfer or operation process, the liquid medicine 111 is prevented from passing through the internal passage of the neck section 13 to flow into or stagnate in an unexpected section of the medicine dispensing device 1, (such as the interior of a second tubular body 20, which will be described hereinafter).

The medicine dispensing device 1 of the present invention further includes a second tubular body 20. The second tubular body 20 is hollow and includes a tube sealing section 21, a compression section 22 and a communication tube section 23 in communication with each other. One end of the tube sealing section 21 is a closed end 211. The closed end 211 is connected with a second handheld section 212. The second handheld section 212 has a flat configuration. One end of the communication tube section 23, which end is distal from the tube sealing section 21, is in communication with the neck section 13. The compression section 22 is disposed between the tube sealing section 21 and the communication tube section 23 in communication therewith, whereby the receiving space 11 is in communication with the interior of the second tubular body 20. At least one body girth (such as outer diameter or girth width) of the compression section 22 is larger than or equal to the tube sealing section 21 and the communication tube section 23.

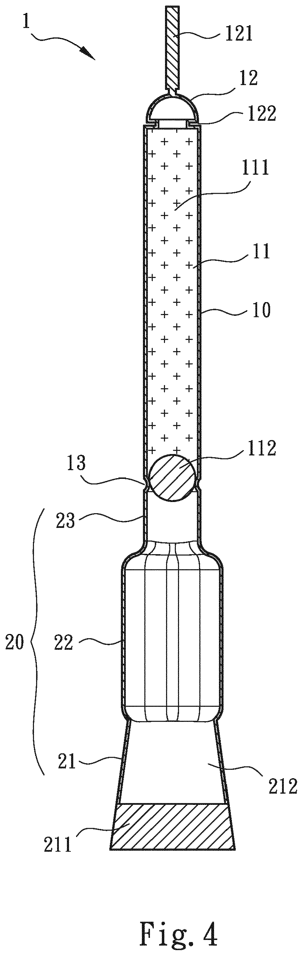

Please refer to Fig. 4. The primary object of the present invention is to prevent the liquid medicine 111 from reversely flowing into or stagnating in the second tubular body 20 of the medicine dispensing device 1 due to the factor of transfer or external force prior to use or in use. This will lead to unsmoothness in dispensing operation of the liquid medicine 111. That is, when the medicine dispensing device 1 is abnormally moved, the stop member 112 is displaced to block the neck section 13 so as to interrupt the passage between the receiving space 11 and the interior of the second tubular body 20. Therefore, the liquid medicine 111 is prevented from flowing into or stagnating in the second tubular body 20.

In addition, in order to facilitate the breakage of the removable section 12 from the first tubular body 10, the radial cross section of the first handheld section 121 and the radial cross section of the second handheld section 212 can intersect each other to contain a proper angle, preferably an angle of 90 degrees. In this case, the first handheld section 121 is normal to the second handheld section 212 (as shown in the drawings) so that the removable section 12 can be more easily broken apart from the first tubular body 10.

The above embodiments are only used to illustrate the present invention, not intended to limit the scope thereof. Many modifications of the above embodiments can be made without departing from the spirit of the present invention.

## Claims

1. A medicine dispensing device (1) comprising:
a first tubular body (10) having an internal receiving space (11), a removable section (12) being disposed at a dispensing end of the first tubular body (10), a liquid medicine (111)being disposed in the receiving space (11), a second tubular body (20) including a tube sealing section (21), a compression section (22) for squeezing out the liquid medicine (111) and a communication tube section (23) in connection with each other, a rear end of the tube sealing section (21) being a closed end (211);and
**characterized in that** a stop member (112) being disposed in the receiving space (11), a neck section (13) being disposed at one end of the first tubular body (10) distal from the removable section (12), at least one body girth of the stop member (112) being larger than the neck section (13);
one end of the communication tube section (23), which end is distal from the tube sealing section (21), being in connection and communication with the neck section (13), the compression section (22) being disposed between the tube sealing section (21) and the communication tube section (23) in communication therewith, whereby the receiving space (11) is in communication with the interior of the second tubular body (20), the stop member (112) serving to block and interrupt the neck section (13) so as to prevent the liquid medicine (111) from passing through the neck section (13) to flow into the second tubular body (20).

2. The medicine dispensing device as claimed in claim 1, wherein a rear end of the removable section (12) has a first handheld section (121), while the second tubular body (20) has a second handheld section (212).

3. The medicine dispensing device as claimed in claim 2, wherein a radial cross section of the first handheld section (121) and a radial cross section of the second handheld section (212) intersect each other to contain an angle of 90 degrees, whereby the first handheld section (121) is normal to the second handheld section (212).

4. The medicine dispensing device as claimed in claim 2 or 3, wherein at least one of the first handheld section (121) and the second handheld section (212) has a flat configuration.

5. The medicine dispensing device as claimed in claim 1, 2 or 3, wherein the body girth of the compression section (22) is larger than or equal to the tube sealing section (21) and the communication tube section (23).

6. The medicine dispensing device as claimed in claim 4, wherein the body girth of the compression section (22) is larger than or equal to the tube sealing section (21) and the communication tube section (23).

7. The medicine dispensing device as claimed in claim 1, 2 or 3, wherein the liquid medicine (111) is at least one of gel, cream and liquid medicine.

8. The medicine dispensing device as claimed in claim 4, wherein the liquid medicine (111) is at least one of gel, cream and liquid medicine.

9. The medicine dispensing device as claimed in claim 5, wherein the liquid medicine (111) is at least one of gel, cream and liquid medicine.

10. The medicine dispensing device as claimed in claim 6, wherein the liquid medicine (111) is at least one of gel, cream and liquid medicine.
